# EUROPEAN PATENT SPECIFICATION

(11) **EP 2 641 551 B1**
(45) Date of publication and mention of the grant of the patent: **18.07.2018**
(21) Application number: 13172807.3
(22) Date of filing: 22.04.2004
(51) Int. Cl.: A61B 17/32

(54) **Devices for reentering a true lumen from a subintimal space**
Vorrichtungen zum Wiedereintreten in ein echtes Lumen aus einem Subintimaraum
Dispositifs pour réintégrer une lumière véritable depuis un espace sous-endothélial

(30) Priority: 22.04.2003 US 421980
(43) Date of publication of application: 25.09.2013
(62) Divisional of application: 04760155.4
(73) Proprietor: Covidien LP, Mansfield, MA 02048 (US)
(72) Inventor: Simpson, John, Woodside, CA California 94062 (US); Rosenthal, Michael, Palo Alto, CA California 95125 (US); Venegas, Gautama, Pleasanton, CA California 94588 (US); Doud, Darren, San Jose, CA California 95130 (US); Ashley, John, San Francisco, CA California 94127 (US)
(74) Representative: Gray, James

(56) References cited:
- US-A1- 2001 000 041
- US-A1- 2002 077 642
- US-A1- 2002 103 459

## Description

### BACKGROUND OF THE INVENTION

The present invention is directed to devices for cutting tissue. In a specific application, the present invention is directed to devices and methods for re-entering the true lumen from a subintimal space such as a dissection plane or so-called "false lumen."

Guidewires and other interventional devices are used to treat vessels and organs using endovascular approaches. A guidewire is typically guided through blood vessels to the treatment site and the device is then advanced over the guidewire. For example, angioplasty and stenting are generally accomplished by first introducing a guidewire to the desired site and then advancing the angioplasty or stent catheter over the guidewire.

When attempting to advance a guidewire or other interventional device through a highly stenosed region or chronic total occlusion (CTO), the guidewire or device may inadvertently enter into the wall of the vessel to create a sub-intimal space. Once in a sub-intimal space, it can be difficult to re-enter the vessel true lumen. Devices for reentering a vessel true lumen from a subintimal location are described in WO 02/45598. Examples of prior art devices are described in US2002/0103459 and US2002/0077642.

### BRIEF SUMMARY OF THE INVENTION

The invention is directed to a device for re-entering a lumen during an endovascular procedure. In a first aspect there is provided an intravascular incising device according to claim 1. The device may be advanced into the subintimal space and energy may then be emitted from the energy emitter to locate the true lumen. In one example, the energy emitter and cutting element may be moved together which exposes the cutting element to cut an access path into the true lumen. In another example, the device may have a bendable tip which is bent while cutting tissue to create the access path or may be bent to direct the device or a guidewire through the access path.

In another example, the rotatable cutting element may be moved from a stored position to a cutting position which exposes over 180 degrees, and even 220 or even 270 degrees of the cutting element relative to the axis of rotation. In another example, the cutter may be gradually exposed as necessary. In still another example, the body of device may be wider along a portion of the device to urge tissue toward the cutting element. The opening may be relatively large and may be open at the distal end and may expose at least part of the cutter at all positions distal to the opening. The open end of the device may permit the tissue to naturally move toward the cutter due to the generally open nature of the distal end.

In still another example, a system for accessing a subintimal space includes a catheter through which the tissue cutting device is advanced. The catheter may be coupled to a fluid source to inject contrast or the like and may also be coupled to a pressure monitor for monitoring pressure to determine when the access path has been created as described in greater detail below.

In a still further example, a method of entering a true lumen from a false lumen during an endovascular procedure is provided. A guidewire may be positioned in the subintimal space. A reentry device may then be advanced over the guidewire to the target location in the subintimal space. The access path may then be created using the reentry device to cut the access path. The same guidewire may then be directed through the access path. The reentry device may have two different openings with the first being used during advancement of the reentry device and a second opening through which the guidewire extends when being directed through the access path. The first opening may be configured to direct the guidewire substantially longitudinal while the second opening directs the guidewire at an angle relative to the longitudinal axis.

These and other aspects of the invention will become apparent from the following description, drawings and claims.

### BRIEF DESCRIPTION OF THE DRAWINGS

FIG. 1 shows a view of the system of the present invention.
FIG. 2 shows a guidewire positioned proximate to a total occlusion.
FIG. 3 shows a subintimal space created adjacent a true lumen by the guidewire.
FIG. 4 shows a reentry device of the present invention advanced over the guidewire to the subintimal space.
FIG. 5 shows a guidewire positioned in the true lumen.
FIG. 6 shows the reentry device with the cutting element in a stored position.
FIG. 7 shows the reentry device with the cutting element in a cutting position.
FIG. 8 is a side view of the reentry device of FIG. 7.
FIG. 9 shows another reentry device with the cutting element in a stored position.
FIG. 10 shows the reentry device of FIG. 9 with the cutting element in a cutting position and the distal portion bent.
FIG. 11 shows the reentry device of FIGS. 9 and 10 with the cutting element advanced to another cutting position which exposes even more of the cutting element and also bends the distal tip further.
FIG. 12 shows another reentry device which has a bendable distal portion.
FIG. 13 shows the reentry device of FIG. 12 with the distal portion bent.
FIG. 14 shows still another reentry device with a cutting element which may be tilted and which does not form part of the present invention.
FIG. 15 shows the reentry device of FIG. 14 with the cutting element tilted to expose more of the cutting element and to move the cutting element through the opening in the body of the device.
FIG. 16 shows the reentry device of FIG. 6 having a junction leading to two separate guidewire outlets with the guidewire positioned in the first outlet during advancement of the device over the guidewire.
FIG. 17 shows the reentry device of FIG. 16 with the guidewire extending through the second outlet for directing the guidewire into the true lumen.
FIG. 18 shows a catheter having a lumen for receiving a guidewire and another lumen which receives the reentry device.
FIG. 19 shows another catheter having a single lumen through which the guidewire and reentry device pass.

### DETAILED DESCRIPTION OF THE INVENTION

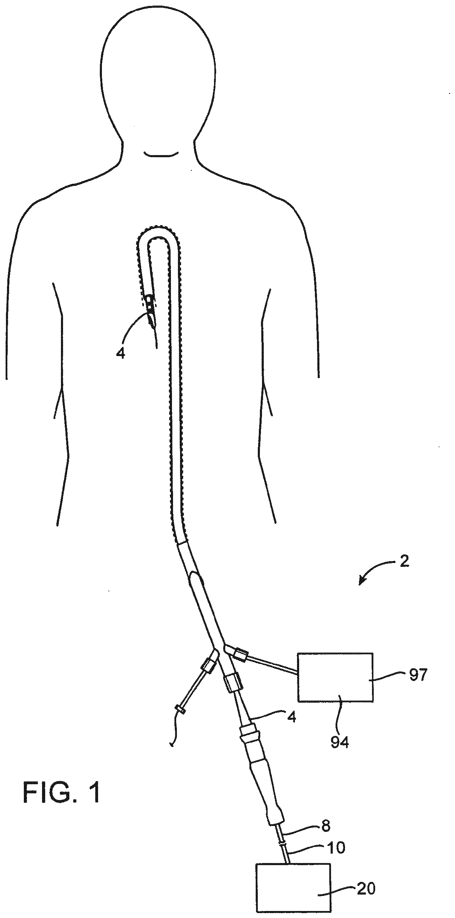

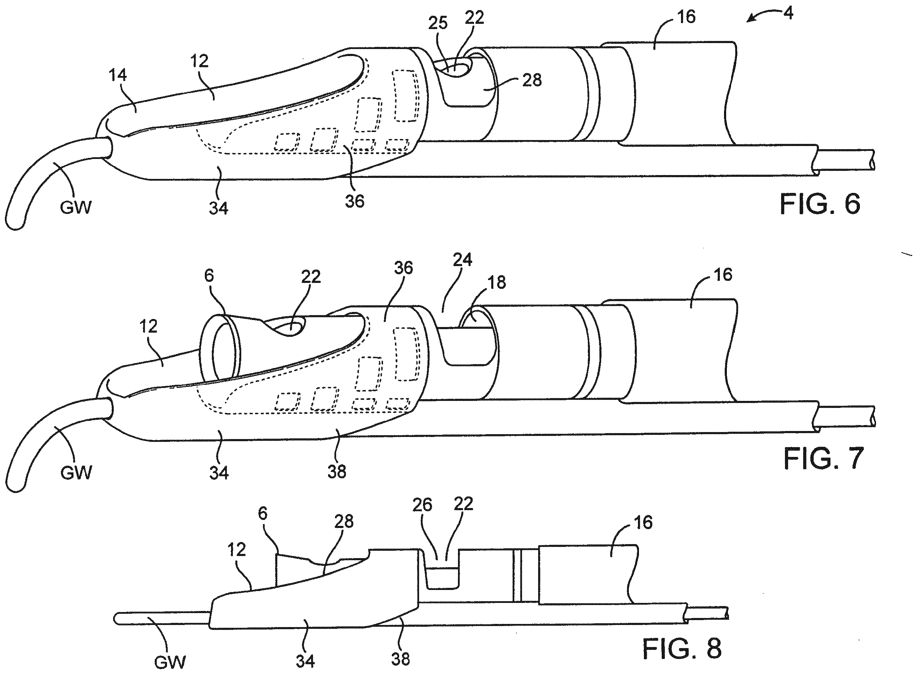

Referring to FIGS. 1-8, a system 2 and device 4 for reentering a true lumen from a subintimal space, dissection plane or so-called false lumen is shown. The device 4 includes a cutting element 6 coupled to a torque transmitting element 8, such as a wire 10, which rotates the cutting element 6. The device 4 has an opening 12 at a distal end 14 with the cutting element 6 movable between a stored position (FIG. 6) and a cutting position (FIGS. 7 and 8) which exposes the cutting element 6. The cutting element 6 may be any suitable cutting element 6 such as the cutting element 6 described in patents incorporated by reference above. The cutting element 6 has a circular cutting edge which has a diameter of about 1 mm although any suitable size may be used depending upon the particular application. The cutting element 6 may also be any other type of cutter such as a laser, ultrasound, RF or other type of cutter without departing from various aspects of the present invention.

The device 4 has a flexible body 16 to navigate through blood vessels or other body lumens to a target location. The body 16 may be made of any suitable material as is known in the art such as Pebax. The torque transmitting element 8 extends through a lumen 18 in the body 16. The body 16 may have more lumens for various reasons such as introduction of fluids, such as contrast, or for delivery of another device 4 such as a guidewire or interventional device. The torque transmitting element 8 is coupled to a driver 20 which rotates the torque transmitting element 8 at a variable or fixed speed.

The device 4 may also have an energy emitting element 22, such as an ultrasound element 25, which emits (and may receive) energy to determine the location of the true lumen as explained below. The energy emitting element 22 is coupled to the cutting element 6 so that the energy emitting element 22 and cutting element 6 are rotated together. The cutting element 6 is in the stored position when locating the true lumen so that the cutting element 6 is not exposed and will not cut or damage tissue. The energy emitting element 22 is positioned adjacent a window 24 which may be a side opening 26 or may simply be a portion of the sidewall which transmits a sufficient amount of the energy therethrough. Any suitable energy emitting element 22 may be used such as the ultrasound emitting element available from Boston Scientific and is marketed under the name Atlantis(TM). The cutting element 6 may be mounted to a collar which is then mounted to an ultrasound element holder 28 or the cutting element 6 may be integrally formed with the ultrasound element holder 28.

The device 4 has an atraumatic tip 34 which is relatively flexible to prevent damaging tissue. The tip 34 may be a separate piece laminated or glued to the body 16. The tip 34 is preferably made out of a relatively soft, flexible material, such as tecothane, and may be used for blunt dissection as necessary. A reinforcing element 36 is encapsulated in the tip 34 to help the tip 34 maintain it's general shape. The tip 34 may also have one or more guidewire lumens 38 or any of the guidewire features described herein.

The opening 12 in the distal portion may be designed to expose over 180 degrees of the cutting element 6 and may even expose 220 degrees or even 270 degrees of the cutting element 6 as defined by the axis of rotation. This provides advantages over WO02/45598 which does not expose much of the cutting element 6 and requires invagination of the tissue within the opening to cut tissue. In another aspect of the invention, the cutting element 6 is gradually exposed. For example, the cutting element 6 may be gradually exposed from 180-220 degrees or even 200-270 degrees. As explained below, this feature provides the user with the ability to change the amount of cutter 6 that is exposed depending upon the tissue thickness between the subintimal location and true lumen. The term opening 12 and amount of exposure of the cutting element 6 are defined by the outer bounds of the opening 12 and the axis of rotation. Referring to FIGS. 7 and 8, the cutting element 6 is exposed relative to the outer bounds of the opening 12 due to the relatively open distal end.

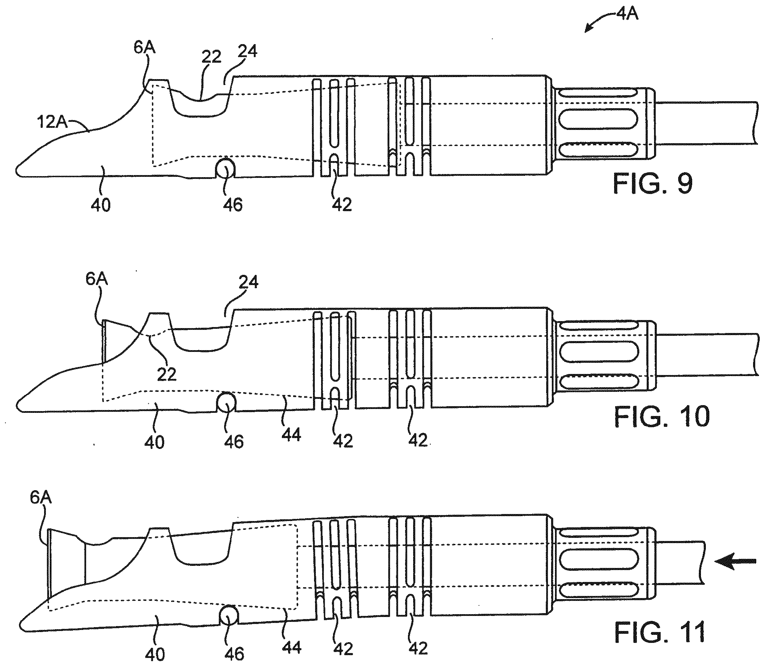

Referring to FIGS. 9-11, another device 4A for reentering a true lumen from a subintimal location is shown wherein the same or similar reference numbers refer to the same or similar structure. The device 4A also has an opening 12A at the distal end to expose the cutting element 6A. FIG. 9 shows the cutting element 6A in a stored position, FIG. 10 shows the cutting element 6A in a first cutting position and FIG. 11 shows the cutting element 6A in a second cutting position which further exposes the element 6A. The device 4A also has the window 24 through which the energy emitting element 22, such as the ultrasound element, may emit energy when the cutting element 6A is in the stored position.

A distal portion 40 of the body can bend or articulate to further expose the cutting element 6A and to move the cutting element 6A toward true lumen. The body has slots 42 formed therein to increase the flexibility of the distal portion 40. The cutting element 6A has a surface 44 which engages a lip 46 on the body. As the cutting element 6A is advanced, the interaction between the surface 44 and lip 46 causes the distal portion 40 to deflect. Bending the distal portion 40 can be helpful in moving the cutting element 6A toward the tissue and may also expose more of the cutting element 6A. As also explained below, the tip 40 may also be bent to direct the device 4A itself or a guidewire into the true lumen. The cutting element 6A may also be gradually exposed as the cutting element 6A moves distally and may be gradually exposed in the same manner described above.

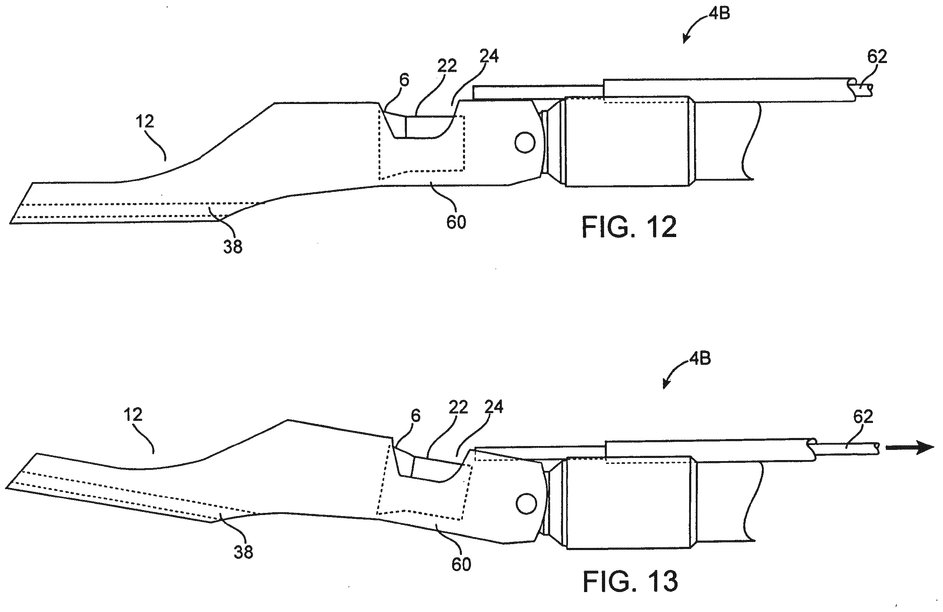

Referring to FIGS. 12 and 13, another reentry device 4B is shown which has a distal portion or tip 60 which bends or articulates. The tip 60 may be articulated and actuated in any suitable manner. For example, the tip 60 may be bent upon longitudinal movement of the cutting element 6 (as shown above) or a separate actuator, such as a pull wire 62, may be used. As can be appreciated from FIG. 13, the tip 60 is bent or articulated to move the cutting element 6 toward the true lumen and to expose more of the cutting element 6. The device 4B may also be bent to direct the device 4B itself or another device or guidewire through the guidewire lumen 38 to the access path into the true lumen as described further below.

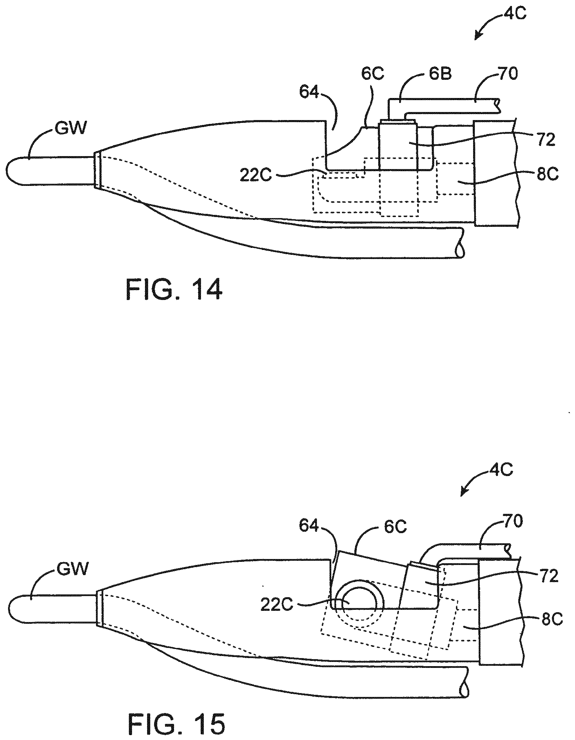

Referring to FIGS. 14 and 15, still another device 4C for cutting tissue is shown wherein the same or similar numbers refer to the same or similar structure. The device 4C includes a cutting element 6C, an energy emitting element 22C and a torque transmitter 8C for rotating the elements. The device 4C has an opening 64 along one side. The cutting element 6C is contained within the opening 64 in the stored position of FIG. 14 and extends out of the opening 64 in the cutting position of FIG. 15. The cutting element 6C is moved out of the window 24 using an actuator 68, such as a wire 70, which tilts a bearing 72 supporting the shaft of the rotatable cutting element 6C. Of course, any other suitable structure may be used to move the cutting element 6C outside the opening 64 such as those described in U.S. Pat. No. 6,447,525 which is hereby incorporated by reference. Furthermore, the cutting element 6C may be moved out of the opening 64 by bending the distal portion or tip as described herein.

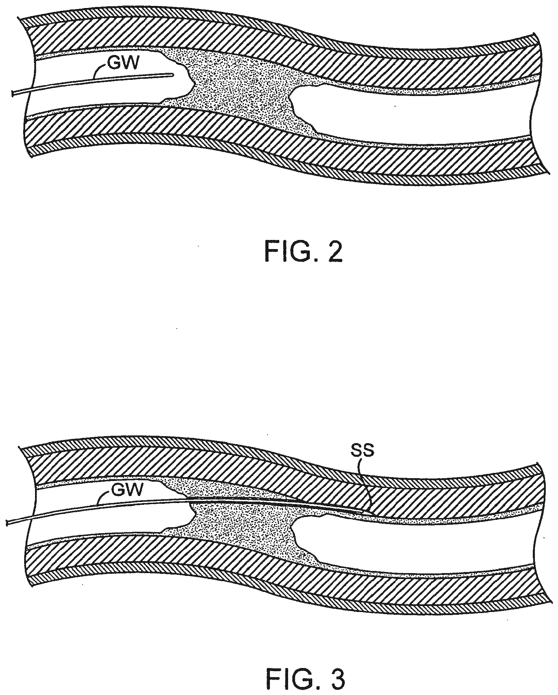

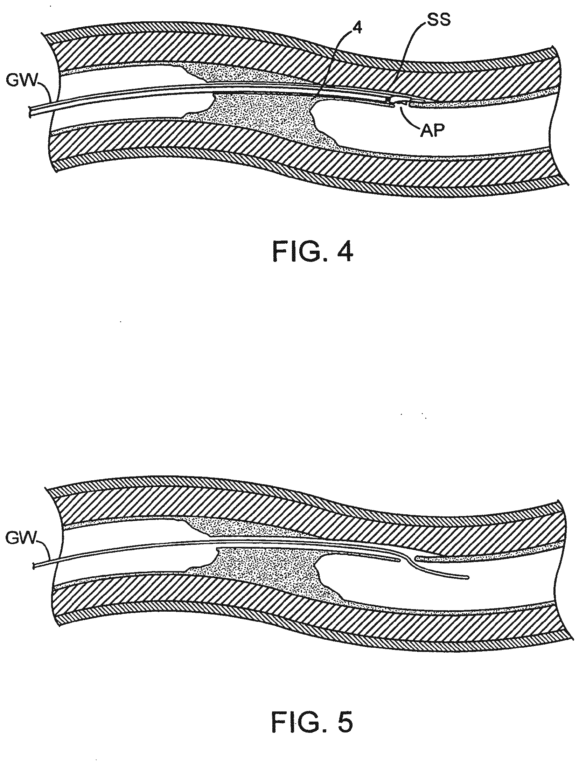

Use of the devices 4, 4A-C is now described with reference to the device 4 although it is understood that any of the devices 4, 4A-C may be used. As mentioned above, the device 4 may be used to perform any suitable procedure to cut from one location to another in the body such as a procedure to reenter a true lumen. The device 4 is initially advanced to a position within a subintimal space SS. As described above, the subintimal space SS may be inadvertently created during an endovascular procedure with a guidewire GW or other device creating the subintimal space SS as shown in FIGS. 2 and 3. The device 4 may be introduced over the same guidewire GW or device which created the subintimal space SS as shown in FIGS. 4 and 5. Of course, the device 4 may also be advanced over the guidewire GW to a position proximate to the subintimal space SS after which the device 4 is then advanced by itself into the subintimal space SS.

After the device 4 is positioned at the appropriate location in the subintimal space SS, the energy emitting element 22 is used to determine the location of the true lumen.

When using the ultrasound element 28, for example, the ultrasound element 28 is rotated while emitting ultrasound energy and the energy emitted through the window 24 and reflected back through the window 24 is processed as is known in the art. The entire device 4 is rotated within the subintimal space SS to orient the window 24 until the true lumen is located. The angular orientation of the device 4 is then maintained so that the opening 12 and window 24 are directed toward the true lumen.

The cutting element 6 is then moved to the cutting position to expose the cutting element 6. The cutting element 6 may be rotated with the driver 20 during this time so that cutting is initiated as the cutting element 6 is exposed. In another aspect of the invention, the entire device 4 itself may be moved through the subintimal space to cut tissue. This provides advantages over the method of WO 02/45598 which requires invagination of tissue through a window to attempt a cut at one location. If the tissue does not invaginate sufficiently into the window, such as when the tissue is too thick, the device of WO 02/45598 will not be able to cut completely through the tissue to create the access path to the true lumen. The user must then move the device and again attempt to invaginate enough tissue to cut an access path. The present invention provides the ability to move the entire device 4 through the subintimal space to create the access path rather than attempting a cut at a single discrete location as in WO 02/45598. Of course, the device 4 may also be used by moving only the cutting element 6 rather than the entire device 4 without departing from the invention.

The cutting element 6 may also be exposed to varying degrees, as described above, until enough of the cutting element 6 is exposed to cut through to the true lumen. For example, the user may choose to expose half of the cutting element 6 and attempt to create an access path to the true lumen. If an access path is not created, the user may then choose to expose more of the cutting element 6 and again attempt to create an access path. This procedure can be repeated until the access path is formed to the true lumen. The device 4A, 4B may be also have a distal tip or portion 40, 60 which bends to move the cutting element 6 toward the tissue and/or expose more of the cutting element 6 during cutting.

After successfully creating the access path into the true lumen, the device 4 itself or part thereof may be directed toward or through the access path. Referring to FIG. 9-13, for example, the distal portion or tip 40, 60 may be bent to help direct the device 4A, 4B itself or the guidewire GW through the access path.

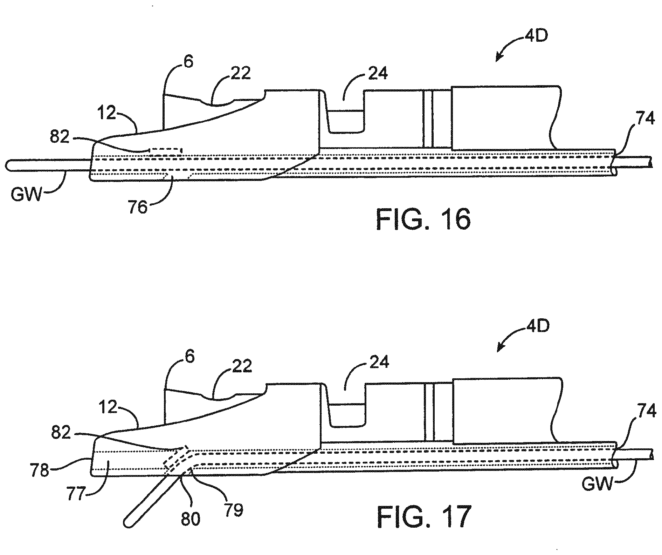

Referring to FIGS. 16 and 17, another device 4D, similar to device 4, is shown which has a guidewire lumen 74 having a junction 76 so the guidewire can be directed through either a first lumen 77 having a first outlet 78 or a second lumen 79 having a second outlet 80. The first outlet 78 directs the guidewire substantially longitudinally for advancing the device 4D over the guidewire to the target area in a conventional manner. The second outlet 80 directs the guidewire at an angle relative to the longitudinal axis, such as 30-75 degrees, to direct the guidewire through the access path into the true lumen.

The junction 76 may include a feature which directs the guidewire into the second outlet 80. Referring to FIG. 17, for example, the junction 76 may include a flap or stop 82 which closes and prevents or inhibits the guidewire from passing through the first outlet 78 after the guidewire has been withdrawn proximal to the junction 76. When the guidewire is advanced again as shown in FIG. 17, the guidewire passes through the second outlet 80 due to the stop 82. The device 4 and/or guidewire GW are then manipulated to direct the guidewire GW through the access path. Although the stop 82 may be provided, the junction 76 may also simply be a relatively open junction 76 with the user manipulating and rotating the guidewire GW to direct the guidewire GW through the desired outlet 78, 80. The device is rotated about 180 degrees after creating the access path to direct the GW through outlet 80 and into the true lumen.

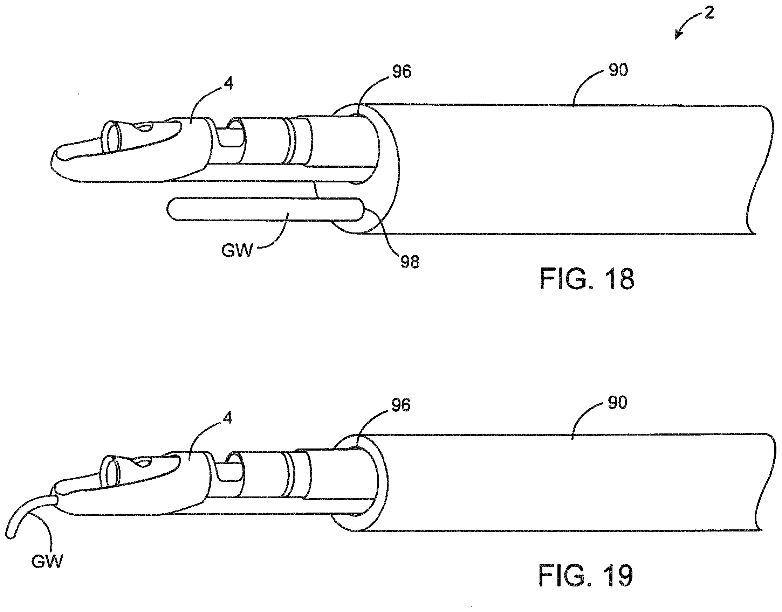

Referring to FIGS. 18 and 19, the system 2 may also include a sheath or catheter 90 which is advanced proximal to the treatment site. The sheath 90 may help provide better control of the guidewire GW and devices 4 of the present invention during manipulation in the subintimal space. The sheath 90 may also used to deliver contrast solution to the treatment site from a source of contrast 97 (see FIG. 1) or may be coupled to a pressure sensor 94. The pressure sensor 94 may be part of the contrast delivery system 97 or may be a separate component. Deliver of contrast and/or pressure monitoring may be used to determine when the access path has been created. [

The sheath 90 may include only one lumen 92 with fluid delivery and pressure sensing being accomplished in the annular space between the device and sheath as shown in FIG. 19. The sheath 90 may also have first and second lumens 96, 98 for separate delivery of the device 4 and guidewire GW. As mentioned above, the devices 4 of the present invention may be advanced over the same guidewire or device that created the subintimal space or may be advanced over another guidewire or even through the sheath 90 by itself.

After accessing the true lumen, another interventional device may be introduced into the true lumen for the intended therapy or procedure. For example, a stent catheter, angioplasty catheter, or atherectomy device may be used to treat the occlusion. The present invention has been described for reentering a true lumen from a subintimal space but, of course, may be used for other purposes to gain access from one space to another anywhere within the body.

The present invention has been described in connection with the preferred embodiments, however, it is understood that numerous alternatives and modifications can be made within the scope of the present invention as defined by the claims.

## Claims

1. An intravascular incising device (4, 4A, 4B, 4D), comprising:
an elongate body (16) having a distal portion (14, 40, 60) with a distal end, an opening (12, 12A) in the distal portion (14, 40, 60), and at least one lumen (18), the body (16) being sufficiently flexible to be advanced through a patient's vasculature to a treatment site;
a torque transmitting element (8) extending through the lumen (18);
a cutting element (6, 6A) coupled to the torque transmitting element (8) and being rotatable about an axis of rotation,
**characterised in that** the opening comprises outer bounds, wherein an amount of exposure of the cutting element is defined by the outer bounds of the opening and the axis of rotation of the cutting element and the cutting element (6, 6A) is movable relative to the opening (12, 12A) in the distal portion (14, 40, 60) to a number of discrete positions to gradually vary the amount of the cutting element (6, 6A) being exposed relative to the opening (12, 12A) as defined by the axis of rotation of the cutting element (6, 6A);
wherein the opening (12, 12A) is in the distal end of the body (16) and the cutting element (6, 6A) is movable distally relative to the body (16) to increase the amount of exposure of the cutting element (6, 6A) relative to the opening (12, 12A) as defined by the axis of rotation of the cutting element (6, 6A).

2. The device of claim 1, wherein:
the cutting element (6, 6A) is distally movable from a stored position in which the cutting element (6, 6A) is not exposed to a working position where the cutting element (6, 6A) is exposed at least 180 degrees relative to the opening (12, 12A) as defined by the axis of rotation of the cutting element (6, 6A).

3. The device of claim 1, wherein:
the cutting element (6, 6A) is distally movable from a stored position in which the cutting element (6, 6A) is not exposed to a working position where the cutting element (6, 6A) is exposed at least 220 degrees relative to the opening (12, 12A) as defined by the axis of rotation of the cutting element (6, 6A).

4. The device of claim 1, wherein:
the cutting element (6, 6A) is distally movable from a stored position in which the cutting element (6, 6A) is not exposed to a working position where the cutting element (6, 6A) is exposed at least 270 degrees relative to the opening (12, 12A) as defined by the axis of rotation of the cutting element (6, 6A).

5. The device of claim 1, wherein:
the cutting element (6, 6A) may be gradually exposed between 180-220 degrees relative to the opening (12, 12A) as defined by the axis of rotation of the cutting element (6, 6A).

6. The device of claim 1, wherein:
the cutting element (6, 6A) may be gradually exposed between 200-270 degrees relative to the opening (12, 12A) as defined by the axis of rotation of the cutting element (6, 6A).

7. The device of claim 1, wherein:
the cutting element (6, 6A) is movable longitudinally relative to the body (16) for moving the cutting element (6, 6A) between the number of discrete positions.

8. The device of claim 2, further comprising:#
an energy emitting element (22) which emits energy to determine the location of a body lumen.

9. The device of claim 8, wherein:
the energy emitting element (22) is coupled to the cutting element (6, 6A) so that the energy emitting element (22) and the cutting element (6, 6A) are rotated together.

10. The device of claim 8, wherein:
The body (16) includes a window (24) the energy emitting element (22) is positioned adjacent the window (24) through which the energy emitting element (22) may emit energy when the cutting element (6, 6A) is in a stored position.

11. The device of claim 1, wherein:
the distal portion (14, 40, 60) is capable of bending to further expose the cutting element (6, 6A).

12. The device of claim 11, wherein:
the distal portion (14, 40, 60) bends upon longitudinal movement of the cutting element (6, 6A).

13. The device of claim 12, further comprising:
a lip (46) on the body (16),
wherein the cutting element (6, 6A) has a surface (44) which engages the lip on the body (16) causing the distal portion (14, 40, 60) to bend upon longitudinal movement of the cutting element (6, 6A).

14. The device of claim 11, further comprising:
an actuator (62) for bending the distal portion (14, 40, 60).

15. The device of claim 14, wherein:
the actuator comprises a pull wire (62).

## Patentansprüche

1. Vorrichtung zum intravaskulären Einschneiden (4, 4A, 4B, 4D), umfassend:
einen länglichen Körper (16), der einen distalen Abschnitt (14, 40, 60) mit einem distalen Ende, einer Öffnung (12, 12A) in dem distalen Abschnitt (14, 40, 60) und zumindest ein Lumen (18) aufweist, wobei der Körper (16) ausreichend flexibel ist, um durch das Gefäßsystem eines Patienten an eine Behandlungsseite eingeführt zu werden;
ein Drehmomentübertragungselement (8), das sich durch das Lumen (18) erstreckt;
ein Schneideelement (6, 6A), das an das Drehmomentübertragungselement (8) gekoppelt ist und um eine Drehachse gedreht werden kann,
**dadurch gekennzeichnet, dass** die Öffnung äußere Begrenzungen umfasst, wobei ein Expositionsausmaß des Schneideelements durch die äußeren Begrenzungen der Öffnung und die Drehachse des Schneideelements definiert ist und das Schneideelement (6, 6A) bezogen auf die Öffnung (12, 12A) in dem distalen Abschnitt (14, 40, 60) in eine Anzahl von getrennten Positionen bewegt werden kann, um das Ausmaß, in dem das Schneideelement (6, 6A) bezogen auf die Öffnung (12, 12A) exponiert ist, wie durch die Drehachse des Schneideelements (6, 6A) definiert, allmählich zu variieren;
wobei sich die Öffnung (12, 12A) an dem distalen Ende des Körpers (16) befindet und das Schneideelement (6, 6A) bezogen auf den Körper (16) distal bewegt werden kann, um das Expositionsausmaß des Schneideelements (6, 6A) bezogen auf die Öffnung (12, 12A), wie durch die Drehachse des Schneideelements (6, 6A) definiert, zu erhöhen.

2. Vorrichtung nach Anspruch 1, wobei:
das Schneideelement (6, 6A) distal aus einer Ruheposition, in der das Schneideelement (6, 6A) nicht exponiert ist, in eine Arbeitsposition bewegt werden kann, in der das Schneideelement (6, 6A) bezogen auf die Öffnung (12, 12A) um zumindest 180 Grad exponiert ist, wie durch die Drehachse des Schneideelements (6, 6A) definiert.

3. Vorrichtung nach Anspruch 1, wobei:
das Schneideelement (6, 6A) distal aus einer Ruheposition, in der das Schneideelement (6, 6A) nicht exponiert ist, in eine Arbeitsposition bewegt werden kann, in der das Schneideelement (6, 6A) bezogen auf die Öffnung (12, 12A) um zumindest 220 Grad exponiert ist, wie durch die Drehachse des Schneideelements (6, 6A) definiert.

4. Vorrichtung nach Anspruch 1, wobei:
das Schneideelement (6, 6A) distal aus einer Ruheposition, in der das Schneideelement (6, 6A) nicht exponiert ist, in eine Arbeitsposition bewegt werden kann, in der das Schneideelement (6, 6A) bezogen auf die Öffnung (12, 12A) um zumindest 270 Grad exponiert ist, wie durch die Drehachse des Schneideelements (6, 6A) definiert.

5. Vorrichtung nach Anspruch 1, wobei:
das Schneideelement (6, 6A) allmählich zwischen 180-220 Grad bezogen auf die Öffnung (12, 12A) exponiert werden kann, wie durch die Drehachse des Schneideelements (6, 6A) definiert.

6. Vorrichtung nach Anspruch 1, wobei:
das Schneideelement (6, 6A) allmählich zwischen 200-270 Grad bezogen auf die Öffnung (12, 12A) exponiert werden kann, wie durch die Drehachse des Schneideelements (6, 6A) definiert.

7. Vorrichtung nach Anspruch 1, wobei:
das Schneideelement (6, 6A) bezogen auf den Körper (16) längs bewegt werden kann, um das Schneideelement (6, 6A) zwischen der Anzahl von getrennten Positionen bewegen zu können.

8. Vorrichtung nach Anspruch 2, ferner umfassend:
ein Energieabstrahlungselement (22), das Energie emittiert, um die Lage eines Körperlumens zu bestimmen.

9. Vorrichtung nach Anspruch 8, wobei:
das Energieabstrahlungselement (22) an das Schneideelement (6, 6A) gekoppelt ist, sodass das Energieabstrahlungselement (22) und das Schneideelement (6, 6A) zusammen gedreht werden.

10. Vorrichtung nach Anspruch 8, wobei:
der Körper (16) ein Fenster (24) beinhaltet, wobei das Energieabstrahlungselement (22) benachbart zu dem Fenster (24) positioniert ist, durch welches das Energieabstrahlungselement (22) Energie emittieren kann, wenn sich das Schneideelement (6, 6A) in einer Ruheposition befindet.

11. Vorrichtung nach Anspruch 1, wobei:
sich der distale Abschnitt (14, 40, 60) biegen kann, um das Schneideelement (6, 6A) weiter zu exponieren.

12. Vorrichtung nach Anspruch 11, wobei:
sich der distale Abschnitt (14, 40, 60) bei einer Längsbewegung des Schneideelements (6, 6A) biegt.

13. Vorrichtung nach Anspruch 12, ferner umfassend:
eine Lippe (46) an dem Körper (16),
wobei das Schneideelement (6, 6A) eine Fläche (44) aufweist, die in die Lippe an dem Körper (16) eingreift, wodurch der distale Abschnitt (14, 40, 60) veranlasst wird, sich bei einer Längsbewegung des Schneideelements (6, 6A) zu biegen.

14. Vorrichtung nach Anspruch 11, ferner umfassend:
einen Aktor (62) zum Biegen des distalen Abschnitts (14, 40, 60) .

15. Vorrichtung nach Anspruch 14, wobei:
der Aktor einen Zugdraht (62) umfasst.

## Revendications

1. Dispositif d'incision intravasculaire (4, 4A, 4B, 4D), comprenant :
un corps allongé (16) ayant une partie distale (14, 40, 60) comportant une extrémité distale, une ouverture (12, 12A) dans la partie distale (14, 40, 60), et au moins une lumière (18), le corps (16) étant suffisamment flexible pour être avancé dans le système vasculaire d'un patient jusqu'à un site de traitement ;
un élément de transmission de couple (8) s'étendant dans la lumière (18) ;
un élément coupant (6, 6A) couplé à l'élément de transmission de couple (8) et pouvant tourner autour d'un axe de rotation,
**caractérisé en ce que** l'ouverture comprend des limites extérieures, dans lequel un niveau d'exposition de l'élément coupant est défini par les limites extérieures de l'ouverture et l'axe de rotation de l'élément coupant et l'élément coupant (6, 6A) peut être déplacé par rapport à l'ouverture (12, 12A) dans la partie distale (14, 40, 60) sur un certain nombre de positions individuelles de façon à faire varier progressivement le niveau selon lequel l'élément coupant (6, 6A) est exposé par rapport à l'ouverture (12, 12A) telle que définie par l'axe de rotation de l'élément coupant (6, 6A) ;
dans lequel l'ouverture (12, 12A) est dans l'extrémité distale du corps (16) et l'élément coupant (6, 6A) peut être déplacé sur le plan distal par rapport au corps (16) de façon à augmenter le niveau d'exposition de l'élément coupant (6, 6A) par rapport à l'ouverture (12, 12A) telle que définie par l'axe de rotation de l'élément coupant (6, 6A).

2. Dispositif selon la revendication 1, dans lequel :
l'élément coupant (6, 6A) peut être déplacé sur le plan distal depuis une position enregistrée dans laquelle l'élément coupant (6, 6A) n'est pas exposé jusqu'à une position de travail où l'élément coupant (6, 6A) est exposé au moins à 180 degrés par rapport à l'ouverture (12, 12A) telle que définie par l'axe de rotation de l'élément coupant (6, 6A).

3. Dispositif selon la revendication 1, dans lequel :
l'élément coupant (6, 6A) peut être déplacé sur le plan distal depuis une position enregistrée dans laquelle l'élément coupant (6, 6A) n'est pas exposé jusqu'à une position de travail où l'élément coupant (6, 6A) est exposé au moins à 220 degrés par rapport à l'ouverture (12, 12A) telle que définie par l'axe de rotation de l'élément coupant (6, 6A).

4. Dispositif selon la revendication 1, dans lequel :
l'élément coupant (6, 6A) peut être déplacé sur le plan distal depuis une position enregistrée dans laquelle l'élément coupant (6, 6A) n'est pas exposé jusqu'à une position de travail où l'élément coupant (6, 6A) est exposé au moins à 270 degrés par rapport à l'ouverture (12, 12A) telle que définie par l'axe de rotation de l'élément coupant (6, 6A).

5. Dispositif selon la revendication 1, dans lequel :
l'élément coupant (6, 6A) peut être exposé progressivement entre 180 et 220 degrés par rapport à l'ouverture (12, 12A) telle que définie par l'axe de rotation de l'élément coupant (6, 6A).

6. Dispositif selon la revendication 1, dans lequel :
l'élément coupant (6, 6A) peut être exposé progressivement entre 200 et 270 degrés par rapport à l'ouverture (12, 12A) telle que définie par l'axe de rotation de l'élément coupant (6, 6A).

7. Dispositif selon la revendication 1, dans lequel :
l'élément coupant (6, 6A) peut être déplacé longitudinalement par rapport au corps (16) pour déplacer l'élément coupant (6, 6A) entre le nombre de positions individuelles.

8. Dispositif selon la revendication 2, comprenant en outre : un élément émettant de l'énergie (22) qui émet de l'énergie pour déterminer l'emplacement d'une lumière corporelle.

9. Dispositif selon la revendication 8, dans lequel :
l'élément émettant de l'énergie (22) est couplé à l'élément coupant (6, 6A) de sorte que l'élément émettant de l'énergie (22) et l'élément coupant (6, 6A) tournent conjointement.

10. Dispositif selon la revendication 8, dans lequel :
le corps (16) inclut une fenêtre (24), l'élément émettant de l'énergie (22) est positionné au niveau adjacent par rapport à la fenêtre (24) au travers de laquelle l'élément émettant de l'énergie (22) peut émettre de l'énergie lorsque l'élément coupant (6, 6A) est sur une position enregistrée.

11. Dispositif selon la revendication 1, dans lequel :
la partie distale (14, 40, 60) peut être courbée pour exposer davantage l'élément coupant (6, 6A).

12. Dispositif selon la revendication 11, dans lequel :
la partie distale (14, 40, 60) est courbée par un mouvement longitudinal de l'élément coupant (6, 6A).

13. Dispositif selon la revendication 12, comprenant en outre :
une lèvre (46) sur le corps (16),
dans lequel l'élément coupant (6, 6A) a une surface (44) qui met en prise la lèvre sur le corps (16), entraînant la courbure de la partie distale (14, 40, 60) par un mouvement longitudinal de l'élément coupant (6, 6A).

14. Dispositif selon la revendication 11, comprenant en outre :
un actionneur (62) pour courber la partie distale (14, 40, 60).

15. Dispositif selon la revendication 14, dans lequel :
l'actionneur comprend un fil de traction (62).
